# EUROPEAN PATENT SPECIFICATION

(11) **EP 1 480 699 B1**
(45) Date of publication and mention of the grant of the patent: **01.10.2008**
(21) Application number: 03739461.6
(22) Date of filing: 10.02.2003
(51) Int. Cl.: A61M 5/32

(54) **GLASS SAFETY SYRINGE AND RELATIVE SAFETY KIT FOR GLASS SYRINGE**
SICHERHEITSSPRITZE AUS GLAS UND SICHERHEITSKIT FÜR GLASSPRITZE
SERINGUE DE SECURITE EN VERRE ET TROUSSE DE SECURITE ASSOCIEE POUR SERINGUE EN VERRE

(30) Priority: 11.02.2002 EP 02425069
(43) Date of publication of application: 01.12.2004
(73) Proprietor: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo
(86) International application number: PCT/EP2003/001271
(87) International publication number: WO 2003/068297

(56) References cited:
- WO-A-01/41841
- US-A- 5 713 873
- US-A- 6 004 296
- US-A- 6 033 387
- US-A1- 2002 004 649

## Description

The present invention refers to a glass syringe, of the disposable type, provided with an automatic safety device to protect the needle once the injection has been performed. The present invention also refers to a relative safety kit that can be applied to a disposable glass syringe.

A syringe generally comprises a cylindrical body open at the rear to receive a plunger. An internally hollow needle is mounted at the head of the syringe body.

Because of health regulations and to avoid transmission of infectious diseases, syringes must generally be used only once and then discarded. For this reason, there is a growing demand for disposable syringes adapted to prevent further use.

Moreover, syringes generally present drawbacks from the point of view of safety. In fact, once the syringe has been used, the needle remains exposed at the head of the syringe body, with the risk of injuries or accidental needle sticks.

To overcome these drawbacks, plastic disposable syringes having safety devices to cover the needle are available on the market.

Patent application PCT WO 99/37345 describes a disposable safety syringe which has a needle covering sleeve mounted axially on the syringe body and slidable from a retracted position in which it leaves the needle exposed to allow injection, to an extended position in which it completely covers the needle, preventing re-use of the syringe and acting as a protection against accidental needle sticks.

Once the injection has been performed, the sleeve is automatically brought into the extracted safety position, by means of an automatic mechanism and without any intervention by the user.

Glass syringes, on the other hand, are generally designed to be able to be reused. In fact, the glass syringe body can be sterilized at high temperature. For this reason, glass Glass syringes, on the other hand, are generally designed to be able to be reused. In fact, the glass syringe body can be sterilized at high temperature. For this reason, glass syringes generally do not provide any safety device for protection of the needle once the injection has been performed.

As is known, prefilled syringes in which the solution is already inside the barrel of the syringe, ready for injection, are widely available on the market. However, there exist some solutions such as vaccines, antidotes and the like which cannot be stored in plastic containers. Consequently, said solutions must necessarily be placed in prefilled glass syringes.

For this reason and for reasons of hygiene and practicality, glass syringes of the disposable type exist on the market. Disposable glass syringes have the drawback of not having a safety device for protection of the needle.

WO 01/41841 discloses a safety system for syringe comprising a supporting sleeve connected to the rear collar of the syringe body and a inner sleeve sliding between the syringe body and the supporting sleeve.

An object of the present invention is to eliminate the prior art drawbacks, providing a glass safety syringe that is practical, versatile, economical and simple to make.

Another object of the present invention is to provide such a glass safety syringe of the disposable type that is able to prevent further attempts at use.

Another object of the present invention is to provide such a glass syringe that is extremely safe and able to prevent accidental injury and tampering after use.

These objects are achieved in accordance with the invention with the characteristics listed in appended independent claims 1 and 9.

Advantageous embodiments of the invention are apparent from the dependent claims.

The syringe according to the invention comprises a glass syringe body hollow on the inside and open at the front and the rear, a plunger slidable inside the syringe body and provided at the rear with a manually operable stem and an injection needle mounted at the fore end of the syringe body.

To make this syringe a safety syringe according to the invention, there is provided a sheath integrally mounted on the glass syringe body and a sleeve slidably mounted on the sheath to pass from a retracted use position in which the needle protrudes foward from the sleeve to be able to perform the injection to an advanced safety position in which the needle is protected by the sleeve.

Locking means and stops are provided reciprocally in the sheath and sleeve such as to lock sleeve in position when it is in the retracted use position and in the advanced safety position. Operating means able to cooperate with the locking means when the plunger is at the end of the injection stroke are fitted to the rear part of the piston stem, to allow the sleeve to be moved from the retracted use position to the advanced safety position.

The advantages of the syringe according to the invention are obvious in that it allows a glass syringe to be made into a safety syringe, by providing the possibility of covering the needle with the sleeve once the injection has been performed.

Further characteristics of the invention will be made clearer by the detailed description that follows, referring to a purely exemplary and therefore unrestrictive embodiment thereof, illustrated in the appended drawings, in which:
Figure 1 is a top plan view of the safety syringe according to the invention, in the position ready for use, with a needle-covering cap mounted at the head of the syringe body;
Figure 2 is an axial sectional view of the safety syringe of Figure 1, in which the syringe body and the piston stem are shown in side view;
Figure 3 is an axial sectional view taken along the sectional plane III-III of Figure 1, in which the piston stem is shown in side view and in the end of injection position and the needle-covering cap has been removed;
Figure 4 is an axial sectional view, like Figure 3, in which the needle is in the safety position;
Figure 5 is a perspective view, illustrating a sheath for covering a syringe body.

The safety syringe according to the invention, denoted as a whole by reference numeral 100, is described with the aid of the figures.

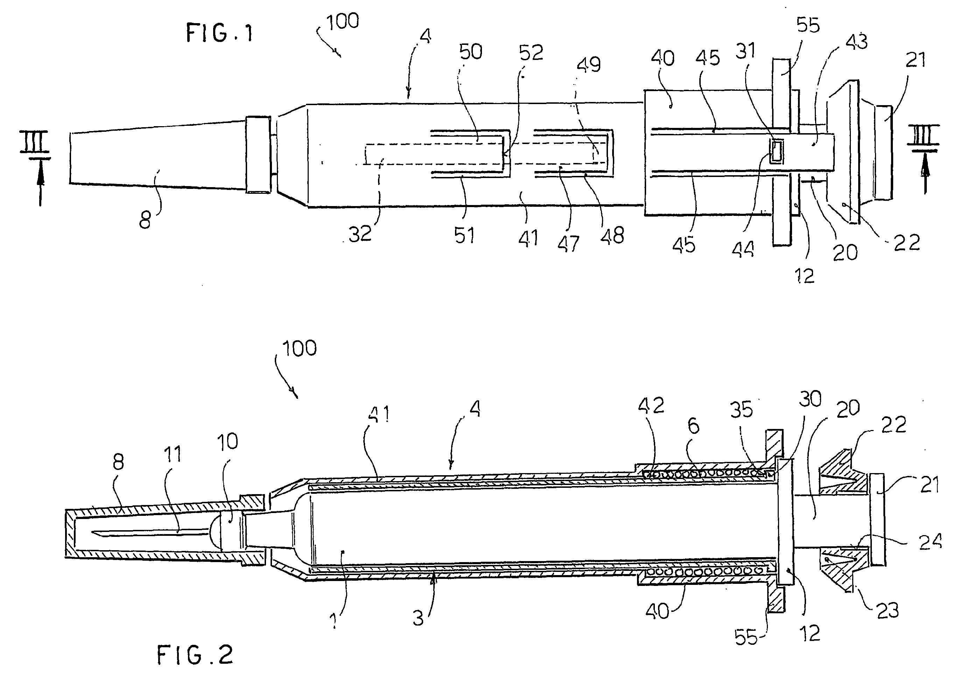

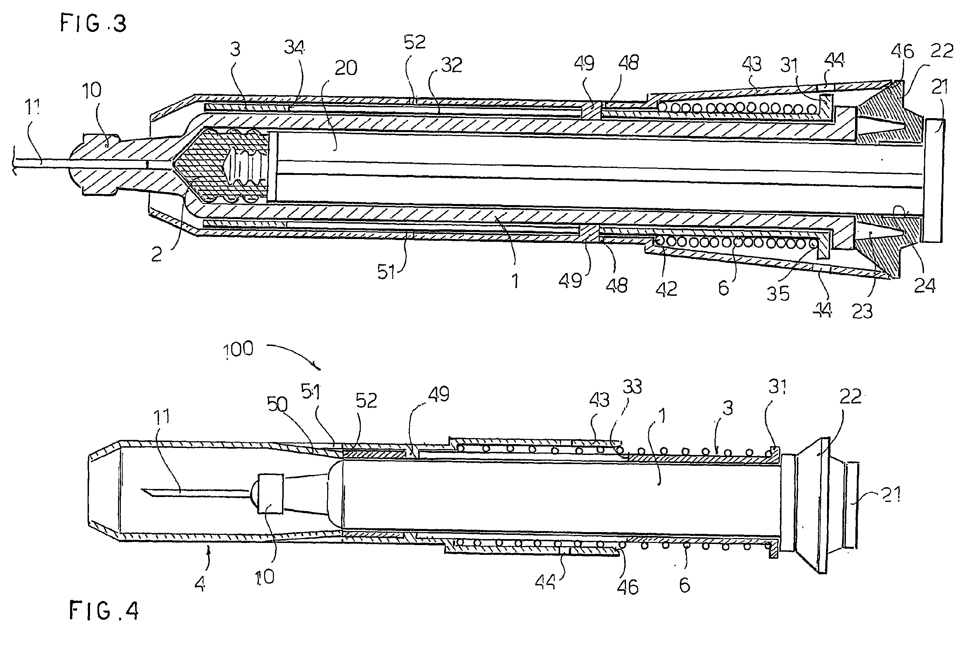

The syringe 100 comprises a syringe body 1 (shown in Figures 2 and 4) made of glass. The syringe body 1 is substantially cylindrical in shape, is hollow on the inside and has a cylindrical chamber open at the front and rear. The body 1 has at its fore end a head 10 into which a needle 11 is sunk. The body 1 has at its rear end a flange 12 that protrudes radially therefrom to facilitate gripping by the user.

As shown in Figure 3, a plunger 2 made of rubber can slide axially with a tight seal in the cylindrical chamber of the syringe body 1. The plunger 2 is supported at the fore end by a stem 20 made of glass or plastic. The stem 20 has at its rear end a flange 21 disposed on the outside of the syringe body 1 to be able to be operated manually by the user.

In the rear part of the stem 20 a frusto conical block 22, disposed around the stem 20, near the flange 21 of the stem, is provided. The frusto conical block 22 has a tapered side surface with a diameter that increases toward the rear part of the stem 20.

The frusto conical block 22 can be formed integrally in the rear part of the stem, or it can be a separate element to apply to the stem 20. In this latter case, the frusto conical block 22 has an axial hole 24 to be forcibly inserted, from the front part of the stem, until it abuts against the rear flange 21 of the stem. In this case, the frusto conical block 22 has an annular groove 23 to be able to yield elastically.

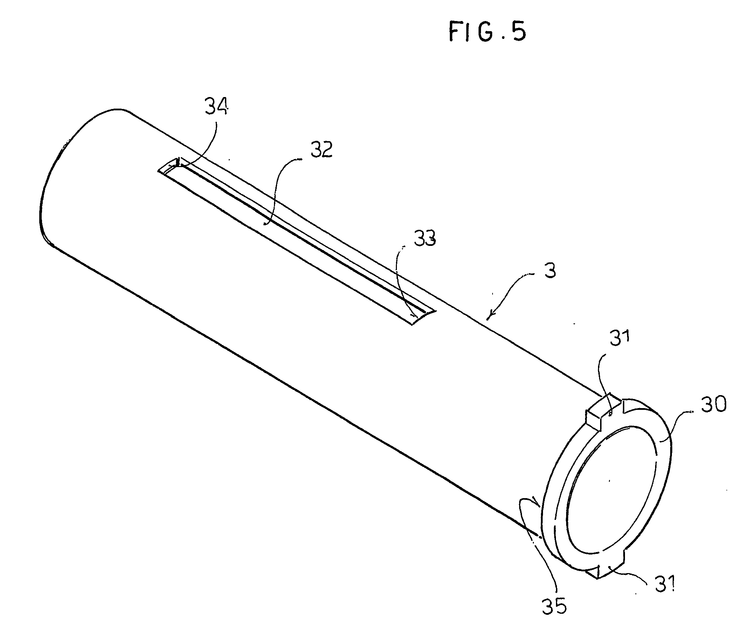

A tubular sheath 3 that almost entirely covers the side surface of the syringe body 1 is mounted on the syringe body 1. As shown also in Figure 5, the sheath 3 comprises at its fore end a collar 30 which protrudes radially outward so as to define an annular abutment surface 35.

Two teeth 31 that protrude radially outward in diametrically opposite directions are provided on the collar 30. Two longitudinal slots 32 disposed in diametrically opposite positions are provided on the side surface of the sheath 3, level with the teeth 31. In this manner, each longitudinal slot 32 defines a rear abutment surface 33 and a front abutment surface 34.

The sheath 3 is made of plastic material and is elastically forced and fixed on the glass body 1. The syringe body 1, with the sheath 3 integral therewith, is mounted axially slidably inside a substantially cylindrical sleeve 4, hollow on the inside. In this manner, the head 10 of the syringe body 1 protrudes forwardly and axially from the sleeve 4 and a needle-covering cap 8 is fitted to the head 10.

The sleeve 4 has a cylindrical rear part 40 with a greater diameter and a cylindrical front part 41 with a smaller diameter, so as to define on the inside an annular abutment surface 42. In this manner, as shown in Figure 2, a helicoidal compression spring 6 which winds around the sheath 3 is interposed between the annular abutment surface 35 of the collar 30 of the sheath 3 and the annular abutment surface 42 of the sleeve 4.

As better shown in Figure 1, two rear tongues 43 are formed in the side wall of the part 40 of the sheath with the largest diameter. The rear tongues 43 are disposed longitudinally in diametrically opposite positions and are flexible. Each rear tongue 43 has a rectangular slot 44 within which the respective tooth 31 of the collar 30 of the sheath snap engages, so as to firmly retain the sheath 3 inside the sleeve 4.

Each rear tongue 43 is defined by two longitudinal parallel notches 45 which reach the end of the part with the greatest diameter 40. In this manner, each rear tongue 43 can bend radially outward. Furthermore, each rear tongue 43 has a rear part which protrudes rearward beyond the rear end of the part with the largest diameter, 40. The rear end of each tongue 43 has a tapered surface 46 able to cooperate with the tapered surface of the frusto conical block 22 of the stem.

At the rear end of the part with the greater diameter 40, a flange 55 protrudes radially to facilitate gripping by the user. The rear flange 55 of the sleeve is not present in the area in which there are the rear tongues 43 so as not to interfere therewith. The flange 55 of the sleeve has in its rear wall an annular abutment surface against which the rear flange 12 of the glass body 1 abuts.

In the lateral part of the part with the smallest diameter 41 of the sleeve two central tongues 47 are formed. The central tongues 47 are disposed longitudinally in diametrically opposite positions and are flexible. Each central tongue 47 is defined by a substantially U-shaped notch 48, so as to be able to bend radially outward.

Each central tongue 47 has an inwardly protruding tooth 49 able to engage in the respective longitudinal slot 32 of the sheath 3. In this manner, when the syringe body 1 with the sheath 3 are inserted inside the sleeve 4, the central tongues 47 are widened and then elastically snap back when the teeth 49 engage in the respective slots 32 in the sheath.

Two front tongues 50 are formed in the side wall of the sleeve part 41 with the smallest diameter. The front tongues 50 are longitudinally disposed in diametrically opposite positions. Each front tongue 50 is defined by a substantially U-shaped notch 51, so as to be able to bend radially outward. Under normal conditions, each front tongue 50 converges inwardly and has at its front end an abutment surface 52.

Operation of the syringe 100 according to the invention will now be described.

With reference to Figures 1 and 2, in an initial condition the spring 6 is compressed between the annular abutment surface 35 of the collar 30 of the sheath 3 and the annular abutment surface 42 of the sleeve 4. The teeth 32 of the sheath are engaged in the slots 44 of the rear tongues 43 of the sleeve avoiding any axial movement of the sheath 2 and body 1 assembly with respect to the sleeve 4. Furthermore, the teeth 49 of the central tongues 47 are in abutment against the front abutment surface 33 of the longitudinal slot 32 of the sheath, avoiding any axial rearward movement of the sheath 3 with respect to the sleeve 4.

The chamber of the body 1 is pre-filled with a solution to be injected. Thus, when the injection is performed and the plunger 2 reaches the end of its stroke in the chamber of the syringe body, as shown in Figure 3, the tapered surface of the frusto conical block 22 cooperates with the tapered surface of the rear end 46 of the rear tongues 43, causing outward radial bending thereof.

Consequently, the teeth 31 of the sheath 3 disengage from the slots 44 of the tongues 43 and the sleeve 4 is no longer firmly constrained to the sheath 3 and syringe body 1 assembly. As a result, as shown in Figure 4, the spring 6 which was compressed is released, lengthening and causing axial forward sliding of the sleeve 4 with respect to the sheath and syringe body assembly, until the teeth 49 of the central tongues 47 of the sleeve abut against the respective rear abutment surfaces 34 of the longitudinal slots 32 of the sheath.

Said axial movement of the sleeve 4 with respect to the sheath and syringe body assembly is guided, since the teeth 49 of the central tongues 47 of the sleeve slide in the respective longitudinal slots 32 of the sheath. Furthermore, when the rear end 52 of the front tongues 50 of the sleeve passes the front end of the sheath 3, the front tongues 50 converge elastically inward.

In this situation, the needle 11 is protected in the safety position inside the sleeve 4. It should be noted that when the syringe 100 is in the safety position, any axial movement of the sleeve 4 with respect to the sheath and syringe body assembly is prevented. In fact, the front end of the sheath 3 abuts against the rear end 52 of the front tongues 50 which are convergent and the rear abutment surfaces 34 of the slots 32 in the sheath are in abutment against the respective teeth 49 of the central tongues 47 of the sleeve.

It is evident that the present invention, besides referring to the safety syringe 100 as described, also refers to a safety kit comprising the sheath 3, the spring 6, the sleeve 4 and the frusto conical block 22, considered as separate pieces which are applied to a glass syringe of the known type to make it a safety syringe.

Numerous variations and changes of detail within the reach of a person skilled in the art can be made to the present embodiment of the invention, without thereby departing from the scope of the invention as set forth in the appended claims.

## Claims

1. A safety syringe (100) comprising:
- a glass syringe body (1) hollow on the inside and open at the front and rear,
- a plunger (2) slidable inside the syringe body (1), said plunger being provided at the rear with a manually operable stem (20) brought out of the syringe body by means of the rear end (21) thereof,
- an injection needle (11) mounted at the front end (10) of the syringe body (1),
- a sheath (3) integrally mounted on said glass syringe body (1),
- a sleeve (4) suitable to pass from a retracted use position in which the needle (11) protrudes forward from said sleeve to be able to perform the injection to an advanced safety position in which the needle (11) is protected by said sleeve (4),
- locking and stop means, mutually provided in said sheath and in said sleeve to lock said sleeve (4) in position when it is in said retracted use position and said advanced safety position, and
- operating means (22) integrally mounted to the rear part of the plunger stem (20) to cooperate with said locking means, when the plunger (2) is at the end of the injection stroke to allow movement of said sleeve (4) from the retracted use position to the advanced safety position,
**characterized in that**
- staid sleeve (4) is slidably mounted on said sheath (3),
- spring means (6) are interposed between said sleeve (4) and said sheath (3),
- said stop means comprise a pair of flexible central tongues (47), longitudinally provided in diametrically opposite positions in the central part of the side wall of said sleeve (4), said central tongues (4) being provided with respective teeth (49) protruding inward to engage in respective longitudinal grooves (32) provided in said sheath (3).

2. A safety syringe (100) according to claim 1, **characterized in that** one end of said spring means is in abutment against an annular abutment surface (42) defined between a larger diameter part (40) and a smaller diameter part (41) of said sleeve and the other end of said spring means is in abutment against an annular abutment surface (35) defined by a collar (30) protruding outward at the fore end of said sheath (3).

3. A safety syringe (100) according to any one of the preceding claims, **characterized in that** said locking means comprise two teeth (31) radially protruding from said sheath, in diametrically opposite directions, to engage in respective slots (44) provided in two rear elastic tongues (43) longitudinally formed in the rear part of the side wall of said sleeve (4).

4. A safety syringe (100) according to claim 3, **characterized in that** each of said rear tongues (43) is defined by two longitudinal parallel notches formed in said side wall of the sleeve, up to the rear end of the sleeve, and the rear end of said tongues (43) protrudes radially beyond the rear end of the sleeve.

5. A safety syringe (100) according to claim 3 or 4, **characterized in that** said operating means comprise a frusto conical block (22) cooperating with said rear tongues (43), when the plunger (2) reaches the end of the injection stroke, in order to make them diverge outward to disengage said teeth (31) of the sheath from said slots (44) of the rear tongues.

6. A safety syringe according to claim 5, **characterized in that** each of said longitudinal grooves (32) in the sheath (3) has a rear abutment surface (33) which abuts against the respective tooth (49) of the central tongues of the sheath when said sheath (4) is in the retracted use position and a front abutment surface (34) that abuts against the respective tooth (49) when said sleeve (4) is in the advanced safety position.

7. A safety syringe (100) according to any one of the preceding claims, **characterized in that** said stop means comprise a pair of flexible rear tongues (50), longitudinally provided in diametrically opposite positions in a rear part of the side wall of said sleeve, said rear tongues (50) being inwardly convergent and having an abutment surface (52) able to abut against the front end of said sheath (3), when the sleeve (4) is in said advanced safety position.

8. A safety syringe (100) according to claim 7, **characterized in that** each of said central tongues (47) and each of said front tongues (50) is defined by a respective substantially U-shaped notch (48, 51) formed respectively in the central part and in the front part of the side wall of said sleeve.

9. A safety kit comprising a sheath (3), spring means (6), a sleeve (4) and operating means (22) to be applied to a syringe to make it a safety syringe
wherein:
- said sheath (3) can be integrally mounted on a syringe body (1) of said syringe, said sleeve (4) is suitable to pass from a retracted use position in which a needle (11) mounted on the front end of the syringe body (1) protrudes forward from said sleeve to be able to perform the injection, to an advanced safety position in which the needle (11) is protected by said sleeve (4),
- said operating means are integrally mountable to the rear part of a plunger stem (20) of the syringe,
- locking and stop means are mutually provided in said sheath (3) and in said sleeve (4) to lock said sleeve in position when it is in said retracted use position and said advanced safety position,
- said operating means cooperate with said locking means, when the plunger is at the end of the injection stroke to allow movement of said sleeve (4) from the retracted use position to the advanced safety position, **characterised in that** :
- said sleeve (4) is slidably mounted on said sheath (3),
- said spring (6) is interposed to act between said sleeve (4) and said sheath (3),
- said stop means comprising a pair of flexible central tongues (47), longitudinally provided in diametrically opposite positions in the central part of the side wall or said sleeve (4), said central tongues (4) being provided with respective teeth (49) protruding inward to engage in respective longitudinal grooves (32) provided in said sheath (3).

10. A safety kit according to claim 9, wherein said locking means comprise two teeth (31) radially protruding from said sheath, in diametrically opposite directions, to engage in respective slots (44) provided in two rear elastic tongues (43) longitudinally formed in the rear part of the side wall of said sleeve (4).

11. A safety kit according to claim 10, **characterized in that** said operating means comprise a frusto conical block (22) cooperating with said rear tongues (43), when the plunger (2) reaches the end of the injection stroke, in order to make them diverge outward to disengage said teeth (31) of the sheath from said slots (44) of the rear tongues,

12. A safety kit according to any one of the claims 9 to 11, **characterized in that** each of said longitudinal grooves (32) in the sheath (3) has a rear abutment surface (33) which abuts against the respective tooth (49) of the central tongues of the sheath when said sheath (4) is in the retracted use position and a front abutment surface (34) that abuts against the respective tooth (49) when said sleeve (4) is in the advanced safety position.

13. A safety kit according to any one of the claims 9 to 12, **characterized in that** said stop means comprise a pair of flexible rear tongues (50), longitudinally provided in diametrically opposite positions in a rear part of the side wall of said sleeve, said rear tongues (50) being inwardly convergent and having an abutment surface (52) able to abut against the front end of said sheath (3), when the sleeve (4) is in said advanced safety position

## Patentansprüche

1. Sicherheitsspritze (100) mit:
- einem hohlen Glasspritzenkörper (1) im Innern und vorne und hinten offen,
- einem verschiebbaren Kolben (2) im Innern des Spritzenkörpers (1), wobei der Kolben hinten mit einem manuell zu bedienenden Stiel (20) versehen ist, der aus dem Spritzenkörper mittels dessen Hinterendes (21) hervorgeholt wird und
- einer Einspritznadel (11), die am Vorderende (10) des Spritzenkörpers (1) befestigt ist,
- einem Gehäuse (3), das einstückig auf dem Glasspitzenkörper (1) befestigt ist.
- einer Hülse (4), die von einer eingezogenen Einsatzposition, in welcher die Nadel (11) vorwärts gerichtet aus der Hülse hervorsteht, geführt werden kann, um in der Lage zu sein, die Einspritzung bis zu einer vorgesetzten Sicherheitsposition, in welcher die Nadel (11) durch die Hülse (4) geschützt ist, durchzuführen.
- Verriegelungs- und Sperrmitteln, die beiderseitig in dem Gehäuse und in der Hülse vorgesehen sind, um die Hülse (4) zu fixieren, wenn sie in der eingezogenen Einsatzposition und der vorgesetzten Sicherheitsposition ist und
- Bedienungsmitteln (22), die einstückig auf dem Hinterteil des Kolbenstiels (20) befestigt sind, um mit den Verriegelungsmitteln zusammenzuwirken, wenn der Kolben (2) am Ende des Einspritzhubs ist, um die Bewegung der Hülse (4) aus der eingezogenen Einsatzposition zur vorgesetzten Sicherheitsposition zu ermöglichen,
**dadurch gekennzeichnet, dass**
- die Hülse (4) verschiebbar auf dem Gehäuse (3) befestigt ist,
- Federmittel (6) zwischen der Hülse (4) und dem Gehäuse (3) eingefügt sind,
- wobei die Sperrmittel ein Paar flexibler mittiger Laschen (47) umfassen, die längslaufend in diametral entgegengesetzten Positionen im Mittelteil der Seitenwand der Hülse (4) vorgesehen sind, wobei die mittigen Laschen (4) mit jeweiligen Zähnen (49) versehen sind, die nach innen hervorstehen, um in jeweilige Längsnuten (32), die in dem Gehäuse (3) vorgesehen sind, einzurücken.

2. Sicherheitsspritze (100) nach Anspruch 1, **dadurch gekennzeichnet, dass** ein Ende der Federmittel an eine ringförmige Angrenzungsfläche (42) grenzt, die zwischen einem breiten Durchmesserteil (40) und einem kleineren Durchmesserteil (41) der Hülse definiert ist, und das andere Ende der Federmittel grenzt an eine ringförmige Angrenzungsfläche (35), die durch einen Bund (30), der nach außen gerichtet am Vorderende des Gehäuses (3) hervorsteht.

3. Sicherheitsspritze (100) nach einem der vorhergehenden Ansprüche, **dadurch gekennzeichnet, dass** die Verriegelungsmittel zwei Zähne (31) umfassen, die radial aus dem Gehäuse in diametral entgegengesetzten Richtungen hervorstehen, um in jeweilige Schlitze (44) einzurücken, die in zwei hinteren elastischen Laschen (43) vorgesehen sind, welche längslaufend im Hinterteil der Seitenwand der Hülse (4) vorgesehen sind.

4. Sicherheitsspritze (100) nach Anspruch 3, **dadurch gekennzeichnet, dass** jede der hinteren Laschen (43) durch zwei parallele Längsnuten in der Seitenwand des Gehäuses bis zum hinteren Ende des Gehäuses definiert ist, und das Hinterende der Laschen (43) steht radial über das Hinterende des Gehäuses hinaus.

5. Sicherheitsspritze (100) nach Anspruch 3 oder 4, **dadurch gekennzeichnet, dass** die Bedienungsmittel einen kegelstumpfartigen Block (22) umfassen, der mit den hinteren Laschen (43) zusammenwirkt, wenn der Kolben (2) das Ende des Einspritzhubs erreicht, um sie nach außen gerichtet divergieren zu lassen, damit sich die Zähne (31) vom Gehäuse aus den Schlitzen (44) der hinteren Laschen lösen.

6. Sicherheitsspritze (100) nach Anspruch 5, **dadurch gekennzeichnet, dass** jede der Längsnuten (32) in dem Gehäuse (3) eine hintere Angrenzungsfläche (33), welche an die jeweiligen Zähne (49) der mittigen Laschen der Gehäuse grenzt, wenn das Gehäuse (4) in der eingezogenen Einsatzposition ist und eine vordere Angrenzungsfläche (34), welche an die jeweiligen Zähne (49) angrenzt, wenn die Hülse (4) in der vorgesetzten Sicherheitsposition ist, aufweist.

7. Sicherheitsspritze (100) nach einem der vorhergehenden Ansprüche, **dadurch gekennzeichnet, dass** die Sperrmittel ein Paar flexibler hinterer Laschen (50) umfassen, die längslaufend in diametral entgegengesetzten Positionen in einem Hinterteil der Seitenwand der Gehäuse vorgesehen sind, wobei die hinteren Laschen (50) nach innen gerichtet konvergent sind und eine Angrenzungsfläche (52) aufweisen, die an das Vorderende des Gehäuses (3) grenzt, wenn die Hülse (4) in der vorgesetzten Sicherheitsposition ist.

8. Sicherheitsspritze (100) nach Anspruch 7, **dadurch gekennzeichnet, dass** jede der mittigen Laschen (47) und jede der vorderen Laschen (50) durch eine jeweilige im Wesentlichen U-förmige Nute (48, 51) definiert ist, die jeweils im Mittelteil und im Vorderteil der Seitenwand der Gehäuse gebildet sind.

9. Sicherheitskit mit einem Gehäuse (3), Federmitteln (6), einer Hülse (4) und Bedienungsmitteln (22), die auf einer Spritze aufzubringen sind, damit sie zu einer Sicherheitsspritze wird,
wobei
- das Gehäuse (3) einstückig auf einem Spritzenkörper (1) der Spritze befestigt werden kann, um aus einer eingezogenen Einsatzposition, in welcher eine Nadel (11), die auf dem vorderen Ende des Spritzenkörpers (1) befestigt ist, vorwärts gerichtet aus dem Gehäuse hervorsteht, geführt zu werden, um in der Lage zu sein, die Einspritzung bis zu einer vorgesetzten Sicherheitsposition, in welcher die Nadel (11) durch die Hülse (4) geschützt ist, durchzuführen,
- die Bedienungsmittel einstückig an das Hinterteil eines Kolbenstiels (20) der Spritze befestigt werden können,
- Verriegelungs- und Sperrmittel beiderseitig in dem Gehäuse (3) und in der Hülse (4) vorgesehen sind, um das Gehäuse zu fixieren, wenn es in der eingezogenen Einsatzposition und der vorgesetzten Sicherheitsposition ist,
- die Bedienungsmittel mit den Verriegelungsmitteln zusammenzuwirken, wenn der Kolben am Ende des Einspritzhubs ist, um die Bewegung der Hülse (4) aus der eingezogenen Einsatzposition zur vorgesetzten Sicherheitsposition zu ermöglichen,
**dadurch gekennzeichnet, dass**:
- die Hülse (4) verschiebbar auf dem Gehäuse (3) befestigt ist,
- die Feder (6) eingefügt ist, um zwischen der Hülse (4) und dem Gehäuse (3) zu wirken,
- die Sperrmittel ein Paar flexible mittige Laschen (47) umfassen, die längslaufend in diametral entgegengesetzten Positionen im Mittelteil der Seitenwand der Hülse(4) vorgesehen sind, wobei die mittigen Laschen (4) mit jeweiligen Zähnen (49) versehen sind, die nach innen hervorstehen, um in jeweilige Längsnuten (32), die in dem Gehäuse (3) vorgesehen sind, einzurücken.

10. Sicherheitskit nach Anspruch 9, wobei die Verriegelungsmittel zwei Zähne (31) umfassen, die radial aus den Zähnen in diametral entgegengesetzten Richtungen hervorstehen, um in jeweilige Schlitze (44) einzurücken, die in zwei hinteren elastischen Laschen (43) vorgesehen sind, welche im Hinterteil der Seitenwand der Hülse (4) gebildet sind.

11. Sicherheitskit nach Anspruch 10, **dadurch gekennzeichnet, dass** die Bedienungsmittel einen kegelstumpfförmigen Block (22) umfassen, der mit den hinteren Laschen (43) zusammenwirkt, wenn der Kolben (2) das Ende des Einspritzhubs erreicht, um sie nach außen gerichtet divergieren zu lassen, damit sich die Zähne (31) vom Gehäuse aus den Schlitzen (44) der hinteren Laschen lösen

12. Sicherheitskit nach einem der Ansprüche 9 bis 11, **dadurch gekennzeichnet, dass** jede der Längsnuten (32) in dem Gehäuse (3) eine hintere Angrenzungsfläche (33), welche an die jeweiligen Zähne (49) der mittigen Laschen des Gehäuses grenzt, wenn das Gehäuse (4) in der eingezogenen Einsatzposition ist und eine vordere Angrenzungsfläche (34), welche an die jeweiligen Zähne (49) angrenzt, wenn das Gehäuse (4) in der vorgesetzten Sicherheitsposition ist, aufweist.

13. Sicherheitskit nach einem der Ansprüche 9 bis 12, **dadurch gekennzeichnet, dass** die Sperrmittel ein Paar flexibler hinterer Laschen (50) umfassen, die längslaufend in diametral entgegengesetzten Positionen in einem Hinterteil der Seitenwand des Gehäuses vorgesehen sind, wobei die hinteren Laschen (50) nach innen konvergent sind und eine Angrenzungsfläche (52) aufweisen, die an das Vorderende des Gehäuses (3) grenzt, wenn die Hülse (4) in der vorgesetzten Sicherheitsposition ist.

## Revendications

1. Seringue de sécurité (100) comportant :
- un corps de seringue en verre (1) creux sur l'intérieur et ouvert à l'avant et à l'arrière,
- un poussoir (2) pouvant glisser à l'intérieur du corps de seringue (1), ledit poussoir étant équipé à l'arrière d'une tige actionnable manuellement (20) amenée hors du corps de seringue par son extrémité arrière (21), et
- une aiguille d'injection (11) montée à l'extrémité avant (10) du corps de seringue (1),
- une gaine (3) montée en tant que partie intégrante sur ledit corps de seringue en verre (1),
- un manchon (4) apte à passer d'une position d'utilisation rétractée dans laquelle l'aiguille (11) dépasse vers l'avant dudit manchon de manière à être apte à réaliser l'injection jusqu'à une position de sécurité avancée dans laquelle l'aiguille (11) est protégée par ledit manchon (4),
- des moyens de verrouillage et d'arrêt installés mutuellement dans ladite gaine et dans ledit manchon pour verrouiller ledit manchon (4) en position lorsqu'il est dans ladite position d'utilisation rétractée et ladite position de sécurité avancée, et
- un moyen d'actionnement (22) monté en tant que partie intégrante sur la partie arrière de la tige de poussoir (20) et destiné à coopérer avec ledit moyen de verrouillage lorsque le plongeur (2) est au bout de la course d'injection afin de permettre le mouvement dudit manchon (4) de la position d'utilisation rétractée vers la position de sécurité avancée,
**caractérisée en ce que**
- ledit manchon (4) est monté de manière à pouvoir glisser sur ladite gaine (3),
- un moyen à ressort (6) est interposé entre ledit manchon (4) et ladite gaine (3),
- ledit moyen d'arrêt comprend une paire de languettes centrales souples (47) installées longitudinalement dans des positions diamétralement opposées dans la partie centrale de la paroi latérale dudit manchon (4), lesdites languettes centrales (4) étant équipées de dents respectives (49) en saillie vers l'intérieur de manière à s'engager dans des rainures longitudinales respectives (32) pratiquées dans ladite gaine (3).

2. Seringue de sécurité (100) selon la revendication 1, **caractérisée en ce qu'**une extrémité dudit moyen à ressort est en butée contre une surface de butée annulaire (42) définie entre une partie à grand diamètre (40) et une partie à petit diamètre (41) dudit manchon et que l'autre extrémité dudit moyen à ressort est en butée contre une surface de butée annulaire (35) définie par une collerette (30) en saillie vers l'extérieur à l'extrémité avant de ladite gaine (3).

3. Seringue de sécurité (100) selon l'une quelconque des revendications précédentes, **caractérisée en ce que** ledit moyen de verrouillage comprend deux dents (31) en saillie radiale depuis ladite gaine dans des sens diamétralement opposés et destinées à s'engager dans des fentes respectives (44) pratiquées dans deux languettes élastiques arrière (43) formées longitudinalement dans la partie arrière de la paroi latérale dudit manchon (4).

4. Seringue de sécurité (100) selon la revendication 3, **caractérisée en ce que** chacune desdites languettes arrière (43) est définie par deux encoches parallèles longitudinales formées dans ladite paroi latérale du manchon jusqu'à l'extrémité arrière du manchon et que l'extrémité arrière desdites languettes (43) est en saillie radiale au delà de l'extrémité arrière du manchon.

5. Seringue de sécurité (100) selon la revendication 3 ou 4, **caractérisée en ce que** ledit moyen d'actionnement comprend un bloc tronconique (22) coopérant avec lesdites languettes arrière (43) lorsque le poussoir (2) atteint le bout de la course d'injection afin de les faire diverger vers l'extérieur et de désengager lesdites dents (31) de la gaine depuis lesdites fentes (44) des languettes arrière.

6. Seringue de sécurité selon la revendication 5, **caractérisée en ce que** chacune desdites rainures longitudinales (32) de la gaine (3) présente une surface de butée arrière (33) qui bute contre les dents respectives (49) des languettes centrales de la gaine lorsque ladite gaine (4) est en position d'utilisation rétractée et une surface de butée avant (34) qui bute contre les dents respectives (49) lorsque ledit manchon (4) est en position de sécurité avancée.

7. Seringue de sécurité (100) selon l'une quelconque des revendications précédentes, **caractérisée en ce que** ledit moyen d'arrêt comprend une paire de languettes arrière souples (50) installées longitudinalement dans des positions diamétralement opposés dans une partie arrière de la paroi latérale dudit manchon, lesdites languettes arrière (50) étant convergentes vers l'intérieur et présentant une surface de butée (52) apte à buter contre l'extrémité avant de ladite gaine (3) lorsque le manchon (4) est dans ladite position de sécurité avancée.

8. Seringue de sécurité (100) selon la revendication 7, **caractérisée en ce que** chacune desdites languettes centrales (47) et chacune desdites languettes avant (50) est définie par une encoche (48, 51) sensiblement en forme de U formée respectivement dans la partie centrale et dans la partie avant de la paroi latérale dudit manchon.

9. Trousse de sécurité comportant une gaine (3), un moyen à ressort (6), un manchon (4) et un moyen d'actionnement (22) à appliquer à une seringue pour en faire une seringue de sécurité, dans laquelle :
- ladite gaine (3) peut être montée en tant que partie intégrante sur un corps de seringue (1) de ladite seringue (4), ledit manchon (4) est apte à passer d'une position d'utilisation rétractée dans laquelle une aiguille (11) montée à l'extrémité avant du corps de seringue (1) est en saillie vers l'avant depuis ledit manchon afin de réaliser l'injection à une position de sécurité avancée dans laquelle l'aiguille (11) est protégée par ledit manchon (4),
- ledit moyen d'actionnement peut être monté en tant que partie intégrante sur la partie arrière d'une tige de poussoir (20) de la seringue,
- des moyens de verrouillage et d'arrêt sont installés mutuellement dans ladite gaine (3) et dans ledit manchon (4) afin de verrouiller ledit manchon en position lorsqu'il est dans ladite position d'utilisation rétractée et dans ladite position de sécurité avancée,
- ledit moyen d'actionnement coopère avec ledit moyen de verrouillage lorsque le poussoir est au bout de la course d'injection afin de permettre le mouvement dudit manchon depuis la position d'utilisation rétractée à la position de sécurité avancée,
**caractérisée en ce que**
- ledit manchon (4) est monté de manière à pouvoir glisser sur ladite gaine (3),
- ledit ressort (6) est interposé afin d'agir entre ledit manchon (4) et ladite gaine (3),
- ledit moyen d'arrêt comporte une paire de languettes centrales souples (47) installées longitudinalement dans des sens diamétralement opposés dans la partie centrale de la paroi latérale dudit manchon (4), lesdites languettes centrales (4) étant équipées de dents respectives (49) en saillie vers l'intérieur afin de s'engager dans des fentes longitudinales respectives (32) pratiquées dans ladite gaine (3).

10. Trousse de sécurité selon la revendication 9, dans laquelle ledit moyen de verrouillage comprend deux dents (31) en saillie radiale depuis ladite gaine dans des sens diamétralement opposés afin de s'engager dans des fentes respectives (44) pratiquées dans deux languettes élastiques arrière (43) formées longitudinalement dans la partie arrière de la paroi latérale dudit manchon (4).

11. Trousse de sécurité selon la revendication 10, **caractérisée en ce que** ledit moyen d'actionnement comprend un bloc tronconique (22) coopérant avec lesdites languettes arrière (43) lorsque le poussoir (2) atteint le bout de la course d'injection afin de les faire diverger vers l'extérieur et de désengager lesdites dents (31) de la gaine depuis lesdites fentes (44) des languettes arrière.

12. Trousse de sécurité selon l'une quelconque des revendications 9 à 11, **caractérisée en ce que caractérisée en ce que** chacune desdites rainures longitudinales (32) de la gaine (3) présente une surface de butée arrière (33) qui bute contre les dents respectives (49) des languettes centrales de la gaine lorsque ladite gaine (4) est en position d'utilisation finale et une surface de butée avant (34) qui bute contre les dents respectives (49) lorsque ledit manchon (4) est en position de sécurité avancée.

13. Trousse de sécurité selon l'une quelconque des revendications 9 à 12, **caractérisée en ce que caractérisée en ce que caractérisée en ce que** ledit moyen d'arrêt comprend une paire de languettes arrière souples (50) installées longitudinalement dans des positions diamétralement opposées dans une partie arrière de la paroi latérale dudit manchon, lesdites languettes arrière (50) étant convergentes vers l'intérieur et présentant une surface de butée (52) apte à buter contre l'extrémité avant de ladite gaine (3) lorsque le manchon (4) est dans ladite position de sécurité avancée.
